# EUROPEAN PATENT SPECIFICATION

(11) **EP 2 839 808 B1**
(45) Date of publication and mention of the grant of the patent: **29.04.2020**
(21) Application number: 13181177.0
(22) Date of filing: 21.08.2013
(51) Int. Cl.: A61F 2/00

(54) **Expandable surgical mesh**
Ausdehnbares chirurgisches Netz
Filet chirurgical expansible

(43) Date of publication of application: 25.02.2015
(73) Proprietor: King Saud University, 11421 Riyadh (SA)
(72) Inventor: AIDahian, Abdullah Dahian, 11333 Riyadh (SA); AIShomer, Feras Mohammad, 11333 Riyadh (SA)
(74) Representative: Goddar, Heinz J.

(56) References cited:
- EP-A1- 1 800 619
- WO-A1-02/078552
- US-A1- 2003 065 402
- US-A1- 2009 192 530
- US-A1- 2009 275 962

## Description

The present invention relates to an expandable surgical mesh.

Surgical mesh has been used for a long time in many surgical interventions. It is basically a thin delicate film or screen that is manufactured from different compositions and materials with various orientations of the net intersections for more elasticity. It is synthesized in various sizes to be used in different places in the body. The surgical mesh can be applied in a wide variety of wounds or tissues and the main purpose is to aid in supporting and reinforcing the soft tissue in the human body. This is to be done in different surgical approaches.

In surgical procedures there is an increasing trend toward minimally invasive surgery with the utilization of laparoscopic surgery, in which small ports are used to insert the instruments that are manipulated from outside the body under the visualization of a scope "camera". The instruments with different tip types are designated whether to grasp, to cut or to dissect the tissue in order to conduct such interventions.

The surgical mesh can also be inserted through the same ports and is manipulated inside the body using different grabbers that are manipulated from outside the body. Then, the mesh is held using the laparoscopic instruments over the area of defect. Finally, such a delicate mesh is secured into its finalized position with the use of different techniques that in part might involve the use of surgical suture needles and threads or by securing tackers.

The publication by Klosterhalfen et al., Expert Rev. Med. Devices, 2(1) 103-117, (2005) describes the nature and diversity of surgical meshes used together with their relation to abdominal wall defects. A significant part of this report is focussing on the biocompatibility of meshes integrated into the abdominal wall. Also, long-term biocompatibility of surgical meshes and resultant complications are discussed, amongst other things, mesh shrinkage, fibrotic bridging, infections as well as chronic pain.

Mesh shrinkage is a common phenomenon after mesh implantation. Actually, it is not the mesh that shrinks, more precise, it is a reduction of the surface area around the mesh. It has been noted that the body inflammatory response to this object being a foreign material plays a significant role in such either by forming scar tissue or by direct action over the mesh material. The surface area of the mesh placement bed also reduces due to retraction of fibrotic scar tissue. This retraction is a physiological phenomenon that indicates scar maturation that starts with water loss and subsequent reduction of surface area on average up to 60% of the prior placement bed. It is essential, but at the same time hazardous to the repair techniques, if the mesh size was not chosen in a proper way that is appropriate to the size of the body defect. And in fact, with the wide varieties of abdominal wall defects in terms of their size, locations, as well as the underlying anatomy, it is not always easy to conduct.

US 6,666,817 B2 discloses expandable surgical implants and their use that permit a physician to expand, with minimal invasion, a surgical implant that was over-tensioned by a surgeon during implantation or became over-tensioned due to changes in patient's anatomy. The implants may be expanded one or more times by directly or indirectly uncinching one or more expansion loops. These loops can be released either spontaneously by bio-absorption, by the application of external forces like radiofrequency or chemical means or by other surgical interventions. A surgical mesh comprising a resorbable element to be tightened is known from the document WO-A-02/078552.

One significant drawback of this model is that the presence of these loops is not orientated to take the shape of the implanted material into account. Therefore, it consists a limited ability to support multidirectional expansion. Further, the loops might be a problem for laparoscopic instruments holding and handling of the implant. On the other hand, these loops even if the holding unit might be of absorbable material, might represent a defect in the overall design. After placement in the human body, the body usually reacts with inflammatory response to the forging material being implanted. The resulting scar tissue and fibrosis might grow through these loops making the implanted material integrated with the fibrous tissue. This will lead to further shrinkage of the implanted material with limited expansion, even if the holding units are of absorbable material. Also, if the holding unit are of non-absorbable nature, future surgical intervention will be difficult due to the essence of the presence of these loops with fibrous tissue growing through.

It is an object of the present invention to provide an expandable surgical mesh which overcomes the drawbacks of the prior art. Especially, a mesh shall be provided which allows extended expansion of the mesh within a patient's body over the whole range of diverse bodily wall defects and to avoid mesh shrinkage. It is a further object to provide a method for preparing such an expandable surgical mesh.

The first object is achieved by an expandable surgical mesh comprising at least one element of a resorbable material, which element is weaved into and/or attached to the mesh in such a way that, if the element is tightened, at least one area of the mesh is provided having a condensed structure.

A surgical mesh is typically in the form of a web having regular and equidistant structure over the whole area of the mesh. According to the present invention, at least one area of the mesh is provided having a condensed structure. Within the present application, the term "condensed" is to be understood, compared to a non-condensed structure, that this area has a somewhat more condensed structure which can be obtained, for example, by folding, packing or contracting the mesh in this area. This condensed structure can be compared with a curtain wrapped together or as if a mesh is tied on itself.

According to the present invention, any resorbable material utilized in the art of surgery and which can be implanted into a human body can be used. Such resorbable materials are well known to the skilled artisan and include suture threads or membranes.

The element is a multilayer membrane.

More preferably, at least two elements are weaved into and/or attached to the mesh in parallel or different directions.

More preferably, the condensed structure is formed by folded, packed and/or contracted areas.

The different elements are composed of different resorbable materials.

Different areas have a different level of condensing.

Also disclosed but not part of the present invention, is a method for preparing an expandable surgical mesh according to the invention, comprising the steps: a) providing a surgical mesh and at least one element, b) weaving into and/or attaching the element to the mesh, and c) tightening the element thereby condensing at least one area of the mesh.

Surprisingly, it was found that the expandable surgical mesh according to the present invention overcomes many of the drawbacks of the prior art. Especially, the inventive mesh allows extended expansion of the implant mesh within a patient's body over the whole range of diverse bodily wall defects and avoids mesh shrinkage.

In the present invention, the condensed structure is of the same material as the surgical mesh in general and further the mesh is preferably folded, packed or contracted. The expandable surgical mesh can be adapted to all different shapes of implanted mesh with ease of handling and prediction or predetermination of the direction, duration and amount of expansion when the condensed structure is released by resorption of the material of the element. The extended period of time of resorption depends on the composition of resorbable material. All common and human tested meshes that are available on a regular daily basis in surgical field can be utilized in the expandable surgical mesh of the invention.

It is possible to select the best type of surgical mesh for the expandable surgical mesh system with regard to the respective surgical intervention or the patient.

Therefore, it is possible to bypass the limitation of mesh shrinkage obstacle by integrating the mesh which can be used to treat any kind of bodily defect in a way that will assure continuous mesh expansion on the long term.

The invention is now further illustrated by the accompanying figures from which further embodiments, features and advantages may be taken. Figures 1 to 9 and 11 do not relate to embodiments of the invention.
Fig. 1 shows a surgical mesh containing a thread in relaxed state (left) and a suture thread tightened and tied to the mesh (right).
Fig. 2 shows an expandable surgical mesh with two different suture thread materials having different degrees of resorption.
Fig. 3 shows a side view of the expandable surgical mesh with clarification of the mesh with two different tied suture thread materials.
Fig. 4 shows a scheme of an area of condensed mesh structure.
Fig. 5 shows an expandable surgical mesh in which an oval mesh shape is utilized.
Fig. 6 shows an expandable surgical mesh in which the mesh areas of condensed mesh fibers are in an oral shape.
Fig. 7 shows an expandable surgical mesh with two areas of condensed mesh structure in two different axes.
Fig. 8 shows an expandable surgical mesh in which a membrane is utilized for condensation.
Fig. 9 shows a scheme of a mesh area of condensed mesh fibers with point A and point B for attachment of the membrane to the mesh surface.
Fig. 10 shows a model which includes a multilayer membrane.
Fig. 11 shows a scheme in which an expandable surgical mesh is utilized to avoid mesh shrinkage.

Generally, the expandable surgical mesh according to the present invention comprises a usual and human tested mesh and resorbable material that are available on regular daily basis in the surgical field. The surgical mesh is to be manufactured and remodeled in a way that certain mesh fibers are condensed, e.g. folded, packed or contracted, and are secured by material that is resorbable by the patient's body. After implantation of the expandable surgical mesh, the body will resorb the material on an extended period of time depending on the composition of the respective material. The orientation of condensation is to be adjusted depending on the mesh shape and on the required expansion volume for each direction. Due to the resorption, e.g. hydrolysis of the resorbable material by the body, the condensed pursed mesh fibers will be released on an extended period of time and thereby the surgical mesh will expand within the patient's body, particularly, to avoid mesh shrinkage.

The expandable surgical mesh of the present invention can be easily prepared by providing a typical surgical mesh and, for example, a suture thread of resorbable material. This thread is weaved into and/or attached to the mesh. Within the scope of the present invention, the term "weave" means any interconnection of the thread with the surgical mesh and also comprises sewing, stitching or the like.

After having weaved the thread into the surgical mesh, the thread can be tightened resulting in a condensation of the area around the thread. For fixation of this condensed structure, the thread (or the element of resorbable material as such) can be secured at the mesh, for example by tying.

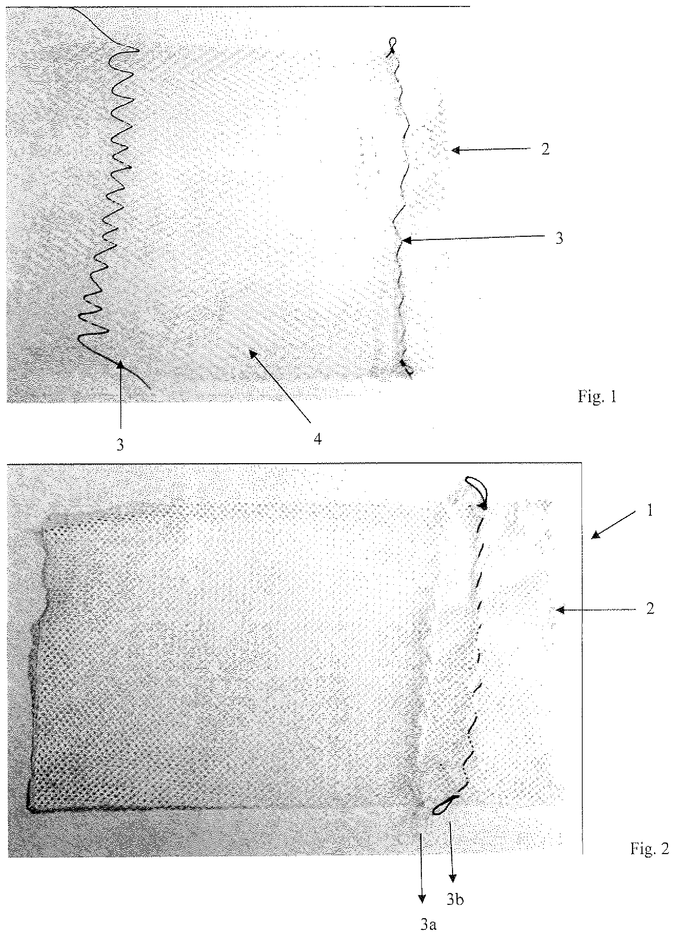

Referring to Fig. 1 an expandable surgical mesh is made of a surgical mesh 2 and a common resorbable suture thread 3. In preferred embodiments, the suture thread 3 is initially running through the mesh 2 in a zigzag fashion in relaxed state (left). The reason for this way of knitting is that when this suture thread 3 is tightened and tied to the mesh 2 it will enforce the mesh fibers 4 to come together in two different axes. Fig. 1 shows as well the expandable surgical mesh 2 in which the suture thread 3 has been tensed and tied over the mesh fibers (right). The tension is translated through the underlying zigzag course of the suture thread 3 into packing of the mesh fibers together with resultant reduction in the surface area that is in two different axes.

Fig. 2 shows a further embodiment in which two different suture thread materials 3a and 3b having different degrees of resorption like fast resorbing and intermediate or long resorbing suture threads are applied. Again, as shown in Fig. 2, the same zigzag course of the suture thread 3 is used with subsequent condensation on the mesh fibers along its axis. Early release of fast resorbing suture thread 3a is anticipated and will allow early expansion of the mesh where needed. This is to be followed by an extended expansion potential with the presence of slow resorbing suture thread 3b.

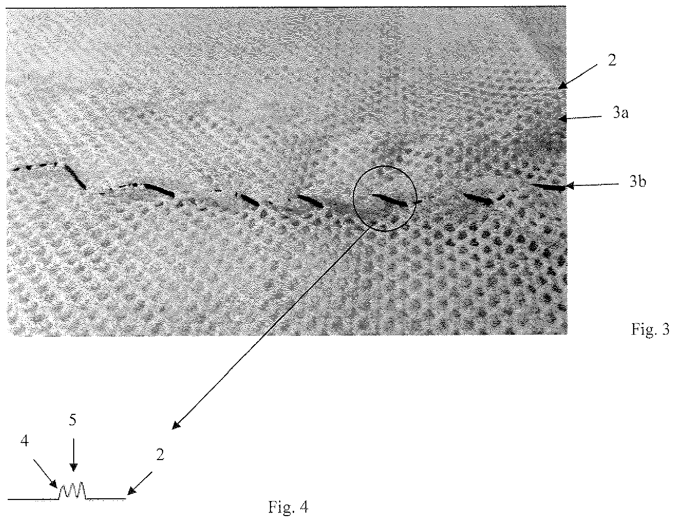

In Fig. 3, a side view with clarification of the surgical mesh 2 with two different tied suture thread materials 3a and 3b is shown. Tensing and tying the suture threads 3a and 3b will result in condensation and packing of the mesh fibers along the long axis of the suture threads 3.

Fig. 4 depicts a condensation area 5 of condensed mesh fibers 4. The degree of condensation will define the later on volume of expansion, in which increased condensation means more mesh fibers are included in the course of the zigzag thread and later on more volume of expansion after the hydrolysis of the suture thread 3 by the body. The range of the overall expansion can range from 2 mm to 5 cm depending on the condensation size. Adjusting the length of each limb of the zigzag course of the suture thread 3 while it's running through the mesh fibers 4 can control the volume condensation of the mesh fibers and eventually the volume of expansion.

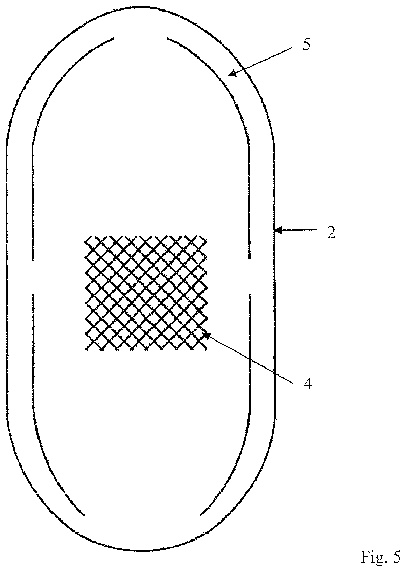

Fig. 5 illustrates the adaptation of the expandable surgical mesh 2 to different mesh shapes and directions of expansion. If the surgical mesh 2 is oval in shape, it can have any one of these areas of condensation 5 of the mesh fibers 4 or all of them in a circular fashion in the same way of suture thread passage or as multiple interrupted ties.

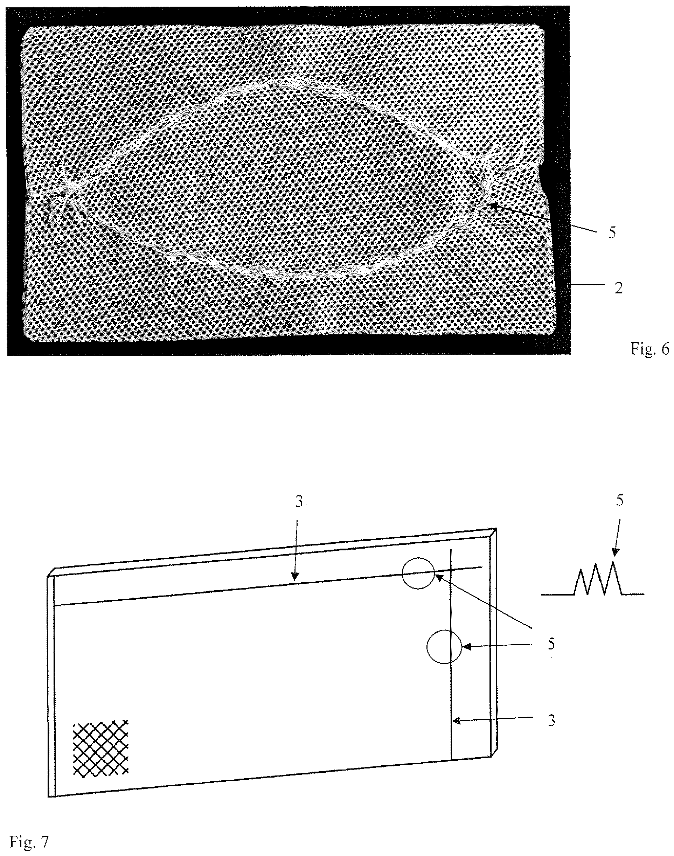

Fig. 6 shows the adaptation of the expandable surgical mesh 2 to different shapes of the tensed and tied mesh areas. The condensed areas 5 of mesh fibers are in an oval shape.

In Fig. 7 an alternative embodiment is shown in which the expandable surgical mesh 2 is applied in condensation areas 5 having two different axes to give an overall multiple axes of condensation. This will allow for more degree of condensation along the long axis of the suture threads 3 as indicated by the arrows. Again, the condensation can be single and/or multiple and can also be vertically, horizontally, or both vertically and horizontal orientated.

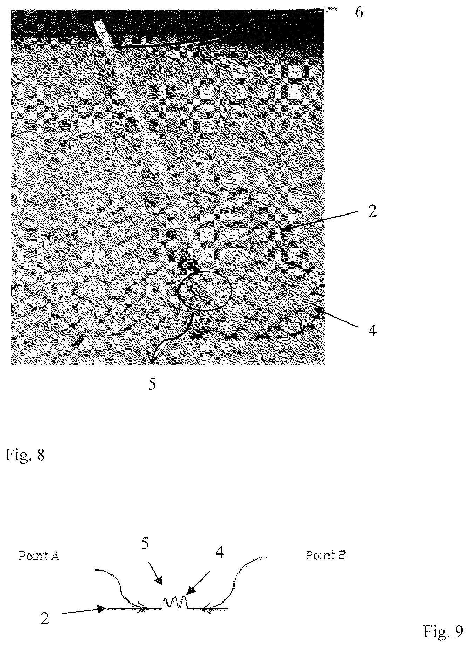

Fig. 8 illustrates embodiments at the expandable surgical mesh 2 in which the resorbable material is a membrane 6 which may be made of the same material as the resorbable suture threads 3 regardless the type and the duration of resorption. This membrane 6 will hold and pack certain areas 5 of the mesh fibers 4 underneath it in a way similar to the use of the suture threads 3. Fig. 9 visualizes the attachment of the membrane 6 to the surface of the surgical mesh 2 at point A and point B with tension in order to induce the overall condensation of the mesh fibers 4.

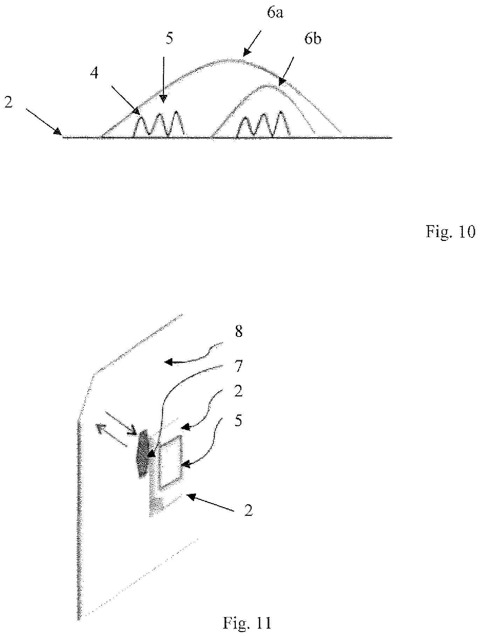

In Fig. 10 the expandable surgical mesh 2 is further expanded to include a multilayer membrane for mesh expansion in which more than one layer of membranes 6 are available. Either each layer is composed from the same material with the same degree of resorption or with a combination of different materials with different rates of resorption. In the latter one, the most external layer of membrane 6a may release its condensed mesh fibers 4 and may expose an underneath layer of another membrane 6b having its own condensed mesh fibers 4 with resultant extended expansion of the overall surgical mesh 2.

In Fig. 11, the expandable surgical mesh 2 is employed to treat a body defect 7 within the body wall 8. As the body will try to aid in mesh shrinking either directly or indirectly by scar tissue maturation, the use of the expandable surgical mesh 2 will help in counteracting this phenomenon on the long term by expanding and further covering a larger gap around the body defect 7.

The features of the present invention disclosed in the specification, the claims and the drawings may both separately in any combination thereof be material for realizing the invention in various forms thereof.

## Claims

1. Expandable surgical mesh comprising an element of a resorbable material, wherein the element is a membrane (6), wherein the membrane (6) is attached to the mesh (2) in such a way, that, if the element is tightened, it will enforce the mesh fibers (4) to come together in two different axes and thereby providing at least two areas of the mesh (2) having a condensed structure and wherein the membrane (6) is a multilayer membrane, wherein the mesh comprises at least two of said elements and wherein the different elements are composed of different resorbable materials and wherein different areas have a different level of condensing.

2. Expandable surgical mesh according to claim 1, wherein at least two elements are weaved into and/or attached to the mesh (2) in parallel or different directions.

## Patentansprüche

1. Expandierbares chirurgisches Netz, das ein Element eines resorbierbaren Materials umfasst, wobei das Element eine Membran (6) ist, wobei die Membran (6) so an dem Netz (2) befestigt ist, dass, wenn das Element gespannt wird, es die Netzfasern (4) dazu zwingt, in zwei verschiedenen Achsen zusammenzukommen und dadurch mindestens zwei Bereiche des Netzes (2) mit einer verdichteten Struktur bereitzustellen, und wobei die Membran (6) eine mehrschichtige Membran ist, wobei das Netz zumindest zwei der besagten Elemente umfasst und wobei die verschiedenen Elemente aus verschiedenen resorbierbaren Materialien zusammengesetzt sind und wobei verschiedene Bereiche einen unterschiedlichen Grad der Verdichtung aufweisen.

2. Expandierbares chirurgisches Netz nach Anspruch 1, wobei zumindest zwei Elemente in das Netz (2) parallel oder in verschiedenen Richtungen eingewebt und/oder daran befestigt sind.

## Revendications

1. Treillis chirurgical expansible comprenant un élément constitué d'un matériau résorbable,
où l'élément est une membrane (6), où la membrane (6) est rattachée au treillis (2) d'une manière telle que si l'élément est resserré, il forcera les fibres du treillis (4) à converger ensemble dans deux axes différents et à produire ainsi au moins deux zones du treillis (2) ayant une structure condensée et où la membrane (6) est une membrane multicouches, le treillis comprenant au moins deux desdits éléments, les différents éléments étant composés de matériaux résorbables différents et les différentes zones ayant un degré de condensation différent.

2. Treillis chirurgical expansible selon la revendication 1, où les au moins deux éléments sont tissés dans le treillis (2) et/ou rattachés à celui-ci dans des directions parallèles ou différentes.
